# EUROPEAN PATENT APPLICATION

(11) **EP 4 372 054 A1**
(43) Date of publication of application: **22.05.2024**
(21) Application number: 22841820.8
(22) Date of filing: 30.05.2022
(51) Int. Cl.: C08L 83/14, A61K 8/89, A61Q 1/00, A61Q 5/00, A61Q 19/00, C08L 83/05, C08L 83/07

(54) **PASTE-LIKE SILICONE COMPOSITION, METHOD FOR PRODUCING SAME, AND COSMETIC**

(30) Priority: 12.07.2021 JP 2021115224
(71) Applicant: SHIN-ETSU CHEMICAL CO., LTD., Tokyo 1000005 (JP)
(72) Inventor: AKABANE Emi, Annaka-shi, Gunma 379-0224 (JP)
(74) Representative: Sonnenhauser, Thomas Martin
(86) International application number: PCT/JP2022/021861
(87) International publication number: WO 2023/286473

(57) **Abstract**

The present invention provides a paste-like silicone composition including (A) crosslinked organopolysiloxane and (B) oil in a liquid state at 25°C. The component (A) includes a hydrosilylated reaction product of (A-1) organohydrogenpolysiloxane having at least two hydrogen atoms bonded to a silicon atom per molecule, and (A-2) alkenyl group-containing organopolysiloxane having at least two alkenyl groups having 2 to 10 carbon atoms and being bonded to a silicon atom per molecule. Octamethylcyclotetrasiloxane, decamethylcyclopentasiloxane, and dodecamethylcyclohexasiloxane contained in the paste-like silicone composition are each less than 2,000 ppm. By these, it is possible to provide a stable paste-like silicone composition that has small changes over time such as plasticization return and emits less uncomfortable odor.

## Description

### TECHNICAL FIELD

The present invention relates to a paste-like silicone composition, a production method for the same, and cosmetics.

### BACKGROUND ART

As a method for stably blending silicone oil, hydrocarbon oil, ester oil, etc. into a cosmetic or a personal care composition, an organosiloxane polymer having crosslinked structure is proposed (Patent Documents 1 to 3). This is known art, the organosiloxane polymer having crosslinked structure (hereinafter, a silicone crosslinked product) is obtained mainly by hydrosilylation-reaction of organohydrogenpolysiloxane and alkenyl-group-containing organopolysiloxane, and is blended in a cosmetic or a personal care composition after being mixed with a part of oil to be used and being made to paste in advance.

However, these prior art does not disclose particularly about low molecular siloxane contained in the organohydrogenpolysiloxane or the alkenyl-group-containing organopolysiloxane to be used. Raw material of the organohydrogenpolysiloxane and the alkenyl-group-containing organopolysiloxane is generally those is hydrolyzed dimethylchlorosilane or siloxanes that contain cyclic siloxane oligomer as a main component, and is manufactured by repartition reaction thereof in the presence of strong acid or a strong base. In this reaction, in many cases, low molecular siloxane having high volatility and hydrocarbon such as hexane and hexene remain as an unreacted material, a byproduct, or an impurity. These low molecular components are not only cause of uncomfortable odor and stimulus but also cause of reduced thickening effect and plasticization return.

Here, plasticization return is a phenomenon in which the viscosity of a paste-like silicone composition increases over time and its properties change. Specific phenomena include a case where a smooth and fluid liquid paste turns into a jelly-like paste free from fluidity, etc. This phenomenon is considered a physical phenomenon, and although it is possible to return the paste to its original state by remixing, etc., it is undeniable that the usability of the paste is significantly reduced. Therefore, in order to improve the quality of the paste-like silicone composition, it is necessary to control the amount of highly volatile low-molecular components.

In the above technical field, the highly volatile components are mainly substances having a molecular weight of 300 or less, and it is known that these can be removed by heating under reduced pressure, thin-film distillation, steam distillation, fractionation using a column, etc. However, it is difficult to remove them after forming a crosslinking structure by hydrosilylation reaction or after forming a paste-like silicone composition by mixing with oil. Besides the production process becomes complicated, when the oil is volatile, the above treatment may also remove the oil, and there is a concern of changing its composition also.

Further, in recent years, some of countries and areas regulates usage and content of a cyclic low molecular siloxane. In these countries and areas, it becomes concern on uses that the low molecular siloxane is remained at high content ratio.

### CITATION LIST

### PATENT LITERATURE

Patent Document 1: JPH01-207354A
Patent Document 2: JPH02-043263A
Patent Document 3: JP2012-162700A

### SUMMARY OF INVENTION

### TECHNICAL PROBLEM

An object of the present invention is to provide a stable paste-like silicone composition which has small changes over time such as plasticization return and emits less uncomfortable odor. It is also an object of the present invention to provide a paste-like silicone composition which contains less cyclic low molecular siloxane regulated in some of countries and areas.

### SOLUTION TO PROBLEM

To achieve the above objects, the present invention provides a paste-like silicone composition comprising:
(A) crosslinked organopolysiloxane; and
(B) oil in a liquid state at 25°C,

wherein octamethylcyclotetrasiloxane, decamethylcyclopentasiloxane, and dodecamethylcyclohexasiloxane contained in the paste-like silicone composition are each less than 2,000 ppm,
and the component (A) comprises a hydrosilylated reaction product of:
   (A-1) organohydrogenpolysiloxane having at least two hydrogen atoms bonded to a silicon atom per molecule; and
   (A-2) alkenyl group-containing organopolysiloxane having at least two alkenyl groups having 2 to 10 carbon atoms and being bonded to a silicon atom per molecule.

Such a paste-like silicone composition has small changes over time such as plasticization return, emits less uncomfortable odor, and is stable.

In this event, the component (A-1) is preferably organohydrogenpolysiloxane represented by the following general formula (1). 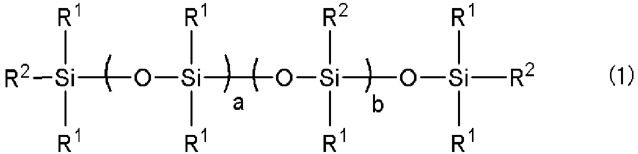 In the general formula (1), R¹ independently represents a group selected from the group consisting of an alkyl group having 1 to 30 carbon atoms, an aryl group having 6 to 30 carbon atoms, and an aralkyl group having 7 to 30 carbon atoms. R² independently represents a group selected from the group consisting of the R¹ and a hydrogen atom provided that two or more R² in one molecule are hydrogen atoms. "a" is an integer satisfying 3≤a≤100,000. "b" is an integer satisfying 1≤b≤50.

Such a paste-like silicone composition can enhance further the advantageous effect of the present invention.

In this event, the component (A-2) is preferably alkenyl group-containing organopolysiloxane represented by the following general formula (2). 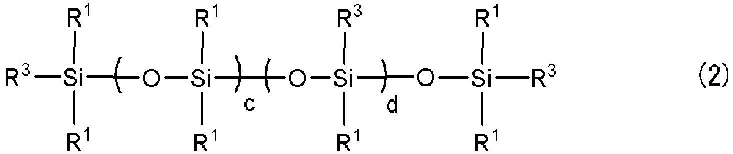 In the general formula (2), R¹ independently represents a group selected from the group consisting of an alkyl group having 1 to 30 carbon atoms, an aryl group having 6 to 30 carbon atoms, and an aralkyl group having 7 to 30 carbon atoms. R³ independently represents a group selected from the group consisting of the R¹ and an alkenyl group having 2 to 10 carbon atoms provided that two or more R³ in one molecule are alkenyl groups. "c" is an integer satisfying 3≤c≤100,000. "d" is an integer satisfying 0≤d≤50.

Such a paste-like silicone composition can enhance further the advantageous effect of the present invention.

In this event, "d" in the general formula (2) preferably satisfies d=0.

Such a paste-like silicone composition can enhance even further the advantageous effect of the present invention.

Further, the present invention provides a method for producing the paste-like silicone composition described in the above, which includes a step of obtaining the component (A) by subjecting the components (A-1) and (A-2) to hydrosilylation reaction in the presence of a catalyst for hydrosilylation reaction, in which the components (A-1) and (A-2) contain octamethylcyclotetrasiloxane, decamethylcyclopentasiloxane, and dodecamethylcyclohexasiloxane, and each content ratio is 2,000 ppm or less in the components (A-1) and (A-2) respectively.

Such a method for producing the paste-like silicone composition is preferable in terms of usability and manufacturing.

In this event, the hydrosilylation reaction is preferably carried out in the presence of 0.01 to 100 mass% of component (B) blended in the paste-like silicone composition.

Such a method for producing the paste-like silicone composition is preferable in terms of usability and manufacturing.

Further, the present invention provides cosmetics containing the paste-like silicone composition described in the above.

Such cosmetics is good in stability and a sensuality, and is environmentally friendly.

### ADVANTAGEOUS EFFECTS OF INVENTION

According to the present invention, by using material containing less amount of a cyclic low molecular siloxane, it is possible to provide a paste-like silicone composition which has small changes over time such as plasticization return, emits less uncomfortable odor, and is stable. Further, it is beneficial also in that it can be used without a concern also in countries and areas where usage and content of a cyclic low molecular siloxane are regulated in recent years. Furthermore, it is possible to provide environment-friendly cosmetics having good stability and sensuality.

### DESCRIPTION OF EMBODIMENTS

As described before, it is desired to develop a paste-like silicone composition which has small changes over time such as plasticization return, emits less uncomfortable odor, and is stable.

As a result of their diligent study of the above problems, the inventor found that uncomfortable odor and plasticization return is prevented by reducing amount of octamethylcyclotetrasiloxane, decamethylcyclopentasiloxane, and dodecamethylcyclohexasiloxane contained in a paste-like silicone composition, and have completed the present invention. Because molecular weights of octamethylcyclotetrasiloxane, decamethylcyclopentasiloxane, and dodecamethylcyclohexasiloxane are 296, 370, and 444 respectively, by control of the amount of these, high volatile components having molecular weight of 300 or less is also sufficiently removed.

That is, the present invention relates to a paste-like silicone composition including:
(A) crosslinked organopolysiloxane; and
(B) oil in a liquid state at 25°C,

wherein octamethylcyclotetrasiloxane, decamethylcyclopentasiloxane, and dodecamethylcyclohexasiloxane contained in the paste-like silicone composition are each less than 2,000 ppm,
and the component (A) includes a hydrosilylated reaction product of:
   (A-1) organohydrogenpolysiloxane having at least two hydrogen atoms bonded to a silicon atom per molecule; and
   (A-2) alkenyl group-containing organopolysiloxane having at least two alkenyl groups having 2 to 10 carbon atoms and being bonded to a silicon atom per molecule.

Hereinafter, the present invention will be described in detail, but the present invention is not limited thereto.

### [Paste-like Silicone Composition]

The inventive paste-like silicone composition includes the following components (A) and (B), and octamethylcyclotetrasiloxane, decamethylcyclopentasiloxane, and dodecamethylcyclohexasiloxane contained in the paste-like silicone composition are each less than 2,000 ppm. In the blow, the components each are described.

### [Component (A)]

The inventive component (A) is crosslinked organopolysiloxane including a hydrosilylated reaction product of:
(A-1) organohydrogenpolysiloxane having at least two hydrogen atoms bonded to a silicon atom per molecule; and
(A-2) alkenyl group-containing organopolysiloxane having at least two alkenyl groups having 2 to 10 carbon atoms and being bonded to a silicon atom per molecule.

Further, the components (A-1) and (A-2) is preferably organopolysiloxane, in which octamethylcyclotetrasiloxane, decamethylcyclopentasiloxane, and dodecamethylcyclohexasiloxane, is contained at 2,000 ppm or less respectively, preferably 1,000 ppm or less, in each of the components (A-1) and (A-2). In each of the components (A-1) and (A-2), the lower the amount of octamethylcyclotetrasiloxane, decamethylcyclopentasiloxane, and dodecamethylcyclohexasiloxane are, the more preferable. The lower limit cannot be determined particularly, but it can be 0 ppm for example.

Structure of the organohydrogenpolysiloxane (A-1) is not particularly limited, but compound represented by the following formula is preferable. 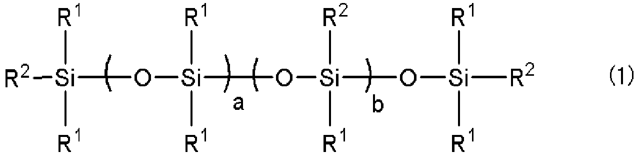 In the above general formula (1), R¹ independently represents a group selected from the group consisting of an alkyl group having 1 to 30 carbon atoms, an aryl group having 6 to 30 carbon atoms, and an aralkyl group having 7 to 30 carbon atoms. R² independently represents a group selected from the group consisting of the R¹ and a hydrogen atom provided that two or more R² in one molecule are hydrogen atoms. "a" is an integer satisfying 3≤a≤100,000. "b" is an integer satisfying 1≤b≤50.

Here, in the above general formula (1), R¹ independently represents a group selected from the group consisting of an alkyl group having 1 to 30 carbon atoms, an aryl group having 6 to 30 carbon atoms, and an aralkyl group having 7 to 30 carbon atoms. The alkyl group has preferably 1 to 10 carbon atoms, more preferably 1 to 6 carbon atoms. The example of the alkyl group can include chain alkyl groups such as a methyl group, an ethyl group, a propyl group, a butyl group, a lauryl group, a myristyl group, a palmityl group, a stearyl group, etc., and cycloalkyl groups such as a cyclopentyl group, a cyclohexyl group, and etc., but the alkyl group is preferably a methyl group or an ethyl group.

The aryl group has preferably 6 to 20 carbon atoms, more preferably 6 to 10 carbon atoms. The example of the aryl group can include a phenyl group, a tolyl group, a xylyl group, etc., but the aryl group is preferably a phenyl group.

The aralkyl group has preferably 7 to 20 carbon atoms, more preferably 7 to 10 carbon atoms. The example of the aralkyl group can include a benzyl group, a phenethyl group, etc., but the aralkyl group is preferably a benzyl group.

R² independently represents a group selected from the group consisting of the R¹ and a hydrogen atom, and two or more R² in one molecule are hydrogen atoms. Two or more and 5 or less of R² in one molecule are preferably hydrogen atoms. Further, in the formula (1), "a" is an integer satisfying 3≤a≤100,000, preferably 5≤a≤1,000, more preferably 10≤a≤500. "b" is an integer satisfying 1≤b≤50, preferably 1≤b≤30, more preferably 2≤b≤10. Further, it is possible to use organohydrogenpolysiloxane satisfying b=0 at the same time.

Further, structure of the alkenyl-group-containing organopolysiloxane (A-2) is not particularly limited, but it is preferable to be represented by the following (2) . 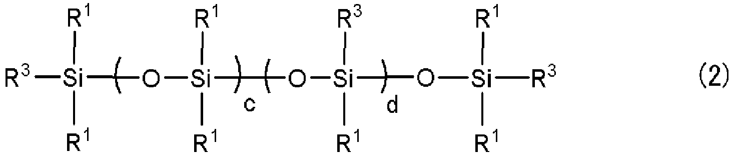 In the general formula (2), R¹ independently represents a group selected from the group consisting of an alkyl group having 1 to 30 carbon atoms, an aryl group having 6 to 30 carbon atoms, and an aralkyl group having 7 to 30 carbon atoms. R³ independently represents a group selected from the group consisting of the R¹ and an alkenyl group having 2 to 10 carbon atoms provided that two or more R³ in one molecule are alkenyl groups. "c" is an integer satisfying 3≤c≤100,000, and "d" is an integer satisfying 0≤d≤50.

Here, in the general formula (2), R¹ is as described in the above, R³ independently represents a group selected from the group consisting of the R¹ and an alkenyl group having 2 to 10 carbon atoms, and two or more R³ in one molecule are alkenyl groups. Examples of the alkenyl groups can include a vinyl group, an allyl group, a propenyl group, a butenyl group, a pentenyl group, a hexenyl group, etc., but a vinyl group is preferable in light of easiness of being hydrosilylated with hydrogen atoms bonded to a silicon atom and good stability. Further, "c" is an integer satisfying 3≤c≤100,000, preferably 5≤c≤10,000, more preferably 10≤c≤1,000. "d" is an integer satisfying 0≤d≤50, preferably 0≤d≤10, more preferably d=0. When d=0, it is possible to enhance advantageous effects of the present invention even further.

Further, in obtaining the component (A), it is also possible to use organohydrogenpolysiloxane having only one hydrogen atoms bonded to a silicon atom in one molecule or organopolysiloxane having only one alkenyl group bonded to a silicon atom in one molecule.

### [Component (B)]

Component (B) is not particularly limited as long as oil is in a liquid state at 25°C, but is preferably liquid oil having a kinematic viscosity at 25°C of 1.0 to 10,000 mm²/s. Examples of preferable oil can include silicone oil, hydrocarbon oil, ester oil, fluorine-based oil, natural animal and plant oil, and semisynthetic oil as follows. In this Description, the kinematic viscosity is a value measured at 25°C according to a method using a Cannon-Fenske viscometer described in JIS Z 8803:2011.

Examples of the silicone oil can include alkyl-modified silicones, such as Dimethicone (INCI), Ethyl Methicone (INCI), Ethyltrisiloxane (INCI), Hexyldimethicone (INCI), etc.; long-chain-alkyl-modified silicones such as Caprylyl Methicone (INCI), etc.; linear or branched organopolysiloxanes such as Phenyl Trimethicone (INCI), Diphenylsiloxy Phenyl Trimethicone (INCI), Diphenyl Dimethicone (INCI), Tetraphenyldimethyldisiloxane (INCI), etc.; cyclic organopolysiloxanes such as Cyclotetrasiloxane (INCI), Cyclopentasiloxane (INCI), Cyclohexasiloxane (INCI), Cycloheptasiloxane (INCI), tetramethyltetraphenylcyclotetrasiloxane, etc.; amino-modified organopolysiloxanes such as Amodimethicone (INCI) and Aminopropyl dimethicone (INCI), etc.; pyrrolidone-modified orgagnopolysiloxanes such as PCA Dimethicone (INCI), etc.; pyrrolidonecarboxylic-acid-modified organopolysiloxanes; amino-acid-modified silicones; and fluorine-modified silicones.

Examples of the hydrocarbon oil can include chain or cyclic hydrocarbon oil. Specific examples can include Olefin Oligomer (INCI), isoparaffin such as C13-14 Isoparaffin (INCI), etc.; alkanes such as Isododecane (INCI), Undecane (INCI), Dodecane (INCI), Isohexadecane (INCI), Hydrogenated Polyisobutene (INCI), Squalane (INCI), Mineral oil (INCI), Coconut Alkane (INCI), C13-15 Alkane (INCI), etc.

Examples of the ester oil can include Diisobutyl Adipate (INCI), dihexyl decyl adipate (labeled name), Diheptylundecyl Adipate (INCI), alkyl glycol monoisostearate such as Isostearyl Isostearate (INCI), Isocetyl Isostearate (INCI), etc.; Trimethylolpropane Triisostearate (INCI), Glycol Diethylhexanoate (INCI), Cetyl Ethylhexanoate (INCI), Trimethylolpropane Triethylhexanoate (INCI), Pentaerythrityl Tetraethylhexanoate (INCI), Cetyl Ethylhexanoate (INCI), octyldodecyl esters such as Octyldodecyl Stearoyl Stearate (INCI), etc.; Oleyl Oleate (INCI), Octyldodecyl Oleate (INCI), Decyl Oleate (INCI), Neopentyl Glycol Diethylhexanoate (INCI), Neopentyl Glycol Dicaprate (INCI), Diisostearyl Malate (INCI), Triethyl Citrate (INCI), Diethylhexyl Succinate (INCI), Amyl Acetate (INCI), Ethyl Acetate (INCI), Butyl Acetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), palmitate esters such as Isopropyl Palmitate (INCI), Ethylhexyl Isopalmitate (INCI), Isocetyl Palmitate, and Hexyldecyl Palmitate (INCI: Isocetyl Palmitate, Hexyldecyl Palmitate), etc.; Cholesteryl Hydroxystearate (INCI), myristate esters such as Isopropyl Myristate (INCI), Octyldodecyl Myristate (INCI), Myristyl Myristate (INCI), etc.; Ethylhexyl Laurate (INCI); Hexyl Laurate (INCI); Dioctyldodecyl Lauroyl Glutamate (INCI); Isopropyl Lauroyl Sarcosinate (INCI); etc.

Among the ester oils, examples of glyceride oil include Triethyl Hexanoin (INCI), Caprylic/Capric Triglyceride (INCI), Coco-Glyceryl (INCI), Caprylic/Capric/Succinic Triglyceride (INCI), (Caprylic/Capric) Glycerides (INCI), etc.

Examples of the fluorine-based oil include perfluoropolyether, perfluorodecaline, perfluorooctane, etc.

Examples of the natural animal or plant oil agent and the semisynthetic oil agent include: Persea Gratissima (Avocado) Oil (INCI), Linum Usitatissimum (Linseed) Seed Oil (INCI), Prunus Amygdalus Dulcis (Sweet Almond) Oil (INCI), perilla oil (labeled name), Olea Europaea (Olive) Fruit Oil (INCI), Torreya Californica (California Nutmeg) Oil (INCI), Cymbopogon Nardus (Citronella) Oil (INCI), Torreya Nucifera Seed Oil (INCI), Kyounin Yu (INCI), Sesamum Indicum (Sesame) Seed Oil (INCI), Oryza Sativa (Rice) Bran Oil (INCI), Camellia Kissi Seed Oil (INCI), Carthamus Tinctorius (Safflower) Seed Oil (INCI), Glycine Soja (Soybean) Oil (INCI), Camellia Sinensis Seed Oil (INCI), Camellia Japonica Seed Oil (INCI), Oenothera Biennis (Evening Primrose) Oil (INCI), RAPE SHUSHI YU (INCI), germ oils such as Zea Mays (Corn) Germ Oil (INCI), Oryza Sativa (Rice) Germ Oil (INCI), Triticum Vulgare (Wheat) Germ Oil (INCI),etc.; natural plant oils such as Persic Oil (labeled name), Elaeis Guineensis (Palm) Oil (INCI), Elaeis Guineensis (Palm) Kernel Oil (INCI), Ricinus Communis (Castor) Seed Oil (INCI), Helianthus Annuus (Sunflower) Seed Oil (INCI), Vitis Vinifera (Grape) Seed Oil (INCI), Simmondsia Chinensis (Jojoba) Seed Oil (INCI), Macadamia Ternifolia Seed Oil (INCI), Limnanthes Alba (Meadowfoam) Seed Oil (INCI), Gossypium Herbaceum (Cotton) Seed Oil (INCI), Cocos Nucifera (Coconut) Oil (INCI), peanut oil, etc.; natural animal oils such as Shark Liver Oil (INCI), Cod Liver Oil (INCI), Fish Liver Oil (INCI), Turtle Oil (INCI), Mink Oil (INCI), Egg Oil (INCI), etc.; and semisynthetic oils such as Hydrogenated Coconut Oil (INCI), Liquid Lanolin (INCI), etc.; etc.

The above oils may be used alone or in combination of a plurality of them. Among them, silicone oil, hydrocarbon oil, or ester oil are preferably be used. Silicone oil or hydrocarbon oil is particularly preferable. Blended amount of component (B) is preferably 1 to 98 mass% of that of the paste-like silicone composition, more preferably 10 to 95 mass%, although the amount depends on the component (A).

Octamethylcyclotetrasiloxane, decamethylcyclopentasiloxane, and dodecamethylcyclohexasiloxane contained in the inventive paste-like silicone composition are each less than 2,000 ppm, preferably less than 1,500 ppm, more preferably less than 1,000 ppm, and further preferably less than 100 ppm. When any of the above cyclic siloxane is 2,000 ppm or more, there is a concern that such a siloxane cannot be used in countries and areas where usage and content of cyclic low molecular siloxane are regulated. The lower the amount of octamethylcyclotetrasiloxane, decamethylcyclopentasiloxane, and dodecamethylcyclohexasiloxane contained in the inventive paste-like silicone composition are, the more preferable. The lower limit cannot be determined particularly, but it can be 0 ppm for example.

### [Cosmetic]

Examples of the cosmetics containing the inventive paste-like silicone composition can include: skin care cosmetics such as milky emulsion, cream, cleansing, pack, massage agent, cosmetic essence, beauty oil, cleaning agent, deodorant, hand cream, lip balm, wrinkle concealer, etc.; makeup cosmetics such as makeup base, concealer, white powder, liquid foundation, oil foundation, blusher, eye shadow, mascara, eyeliner, eyebrow, lipstick, etc.; hair cosmetics such as shampoo, conditioner, treatment, and setting agents, etc.; ultraviolet light-protecting cosmetics such as sunscreen oil, sunscreen emulsion, sunscreen cream, etc.; antiperspirants; etc.

Its properties can be selected from various types, such as liquid, milky lotion, cream, solid, paste, gel, powder, press, multilayer, mousse, spray, stick, etc.

Form of these cosmetics can be selected from various types such as aqueous, oil-based, water-in-oil emulsions, oil-in-water emulsions, non-aqueous emulsions, multi-emulsions such as W/O/W and O/W/O, etc.

Further, the inventive paste-like silicone composition goes beyond the field of cosmetics and can be used for applications such as sealants, coatings, greases, adhesives, defoaming agents, pharmaceuticals, pesticides, and herbicides, and can be used to adjust viscosity of materials and improve their rheological properties.

### [Method for Producing Paste-like Silicone Composition]

A method for producing the inventive paste-like silicone composition is not particularly limited as long as a production method includes a step of obtaining the component (A) by subjecting the components (A-1) and (A-2) to hydrosilylation reaction in the presence of a catalyst for hydrosilylation reaction, and the components (A-1) and (A-2) contain octamethylcyclotetrasiloxane, decamethylcyclopentasiloxane, and dodecamethylcyclohexasiloxane, of which content ratios are each 2,000 ppm or less in the components (A-1) and (A-2) respectively. For example, a paste-like silicone composition can be obtained by kneading of the component (A) obtained by the step of obtaining the component (A) and the component (B) according to a conventional method. It is possible to prevent uncomfortable odor or plasticization return by reducing amount of octamethylcyclotetrasiloxane, decamethylcyclopentasiloxane, and dodecamethylcyclohexasiloxane, which remains in organohydrogenpolysiloxane which is raw material of crosslinking product or alkenyl-group-containing organopolysiloxane.

### [Method for Producing Components (A-1) and (A-2)]

A method for producing the organohydrogenpolysiloxane (A-1) and the alkenyl-group-containing organopolysiloxane (A-2) is not particularly limited. In general, components (A-1) and (A-2) utilize siloxanes as a raw material which contains, as a main component, cyclic and/or acyclic siloxane oligomer obtained by hydrolysis of dimethyl dichlorosilane or by further cracking of the hydrolysis product in the presence of an alkali catalyst such as potassium hydroxide etc., and are obtained by subjecting the raw material to equilibration reaction in the presence of an acid catalyst or an alkali catalyst. By subjecting the obtained polysiloxane to a treatment such as a reduced pressure stripping method, etc., amount of octamethylcyclotetrasiloxane, decamethylcyclopentasiloxane, and dodecamethylcyclohexasiloxane can be reduced each to 2,000 ppm or less.

### [Method for Producing Component (A)]

Reaction of constituting organohydrogenpolysiloxane(A-1) and alkenyl-group-containing organopolysiloxane (A-2) can be carried out by hydrosilylation reaction in the presence of a catalyst for hydrosilylation reaction. By this the component (A) is produced. The catalyst for a hydrosilylation reaction is not particularly limited. For example, in the presence of a catalyst for hydrosilylation reaction such as platinum compounds including chloroplatinic acid, an alcohol-modified chloroplatinic acid, and chloroplatinic acid-vinyl siloxane complex, etc., rhodium compounds, etc., the organohydrogenpolysiloxane (A-1) and the alkenyl-group-containing organopolysiloxane(A-2) may be reacted. In this case, a reaction temperature is not particularly limited, but it is preferably 40 to 120°C. An amount of the added catalyst for a hydrosilylation reaction may be a known effective amount, and is typically 0.1 to 500 ppm, preferably 0.5 to 200 ppm, and more preferably 1 to 100 ppm, in terms of platinum-group metal relative to a total amount of the components (A-1) and (A-2). In this hydrosilylation reaction, a mole ratio of all alkenyl groups / all hydrosilyl groups is not particularly limited, but preferably 1/10 to 10/1, and more preferably 8/10 to 3/1.

The hydrosilylation reaction may be performed without a solvent, or an organic solvent or a volatile dimethylsilicone may be used as necessary. Examples of such an organic solvent can include: aliphatic alcohols such as methanol, ethanol, 2-propanol, butanol, etc.; aromatic hydrocarbons such as benzene, toluene, xylene, etc.; aliphatic or alicyclic hydrocarbons such as n-pentane, n-hexane, cyclohexane, etc.; halogenated hydrocarbons such as dichloromethane, chloroform, carbon tetrachloride, etc.; ketone solvents such as acetone, methyl ethyl ketone, etc.; etc. From the viewpoint of use as cosmetics, solvent-free, ethanol, or 2-propanol is preferable. Examples of the volatile dimethylsilicone can include dimethylpolysiloxane having a kinematic viscosity of 5 mm²/s or less, etc. Such an organic solvent or volatile dimethylsilicone can be removed by heating under a reduced pressure, etc. after the hydrosilylation reaction, as necessary. The hydrosilylation reaction may be performed in the presence of the component (B) liquid oil, and this method is most preferable in terms of usability and manufacturing because other solvent is not used. In this event, the hydrosilylation reaction is preferably carried out in the presence of 0.01 to 100 mass% of blended amount of component (B) blended in the inventive paste-like silicone composition, more preferably carried out in the presence of 0.01 to 90 mass%. For example, including a step of obtaining the component (A) by subjecting the components (A-1) and (A-2) to hydrosilylation reaction in the presence of a catalyst for hydrosilylation reaction, the hydrosilylation reaction can be carried out in the presence of 0.01 to 100 mass% of component (B) blended in the paste-like silicone composition.

### EXAMPLE

Hereinafter, the present invention will be specifically described with reference to Examples and Comparative Examples. However, the present invention is not limited thereto. Note that a method used for each measuring will be shown in the below.

· Contents of cyclic siloxane: After mixing (shaking for 6 hours) 0.5 g of the sample with 10 ml of acetone containing 20 mass ppm of tetradecane as an internal standard, and the mixture are subjected to centrifugation. The acetone layer was collected and subjected to gas chromatography analysis to measure amount of each cyclic siloxane. The results are shown in Table 1.
· Penetration: Measured according to a method described in JIS K 2220:2013.
· Absolute viscosity: Measured by a method using type B rotational viscometer (Product name: TVB10M viscometer, manufactured by Toki Sangyo Co., Ltd) described in JIS K 7117-1:1999.

- Kinematic viscosity: Measured at 25°C by a method using a Cannon-Fenske viscometer described in JIS Z 8803:2011.
- Uncomfortable Odor: A sensory evaluation of odor was conducted by nine panelers, and the most common responses among the following criteria are listed.
   (1) Absent, (2) Somewhat, (3) Present
- Plasticization return: Properties and the next day's properties were judged visually and by hand stirring with a spatula, and were listed using the following expressions.
   (1) Absent, (2) Somewhat, (3) Present

### Example 1

40.9 parts by mass of the organohydrogenpolysiloxane described in (1) of the following Table 1, 10.8 parts by mass of the alkenyl-group-containing organopolysiloxane described in (6) of the following Table 1, 51.8 parts by mass of the dimethylsiloxane (kinematic viscosity of 2 mm²/s) as the oil that is in a liquid state at 25°C, and 0.02 parts by mass of divinyltetramethyldisiloxane solution of 1 mass% chloroplatinic acid as the catalyst for hydrosilylation reaction were added into a reaction vessel, and the mixture was reacted at 70 to 80°C for 2 hours.

Next, the entire amount of the obtained reaction product was sufficiently kneaded with 155 parts by mass of the dimethylsiloxane (kinematic viscosity of 2 mm²/s) using a triple-roller to obtain a smooth paste-like silicone composition having a penetration of 270 in a reactant/liquid oil = 20/80 mass ratio. There was no uncomfortable odor, and no change in properties was observed on the next day. At this time, content ratio, in the paste-like silicone composition, of octamethylcyclotetrasiloxane and decamethylcyclopentasiloxane were each less than 100 ppm, and that of dodecamethylcyclohexasiloxane was 100 ppm.

### Example 2

40.9 parts by mass of the organohydrogenpolysiloxane described in (1) of the following Table 1, 10.0 parts by mass of the alkenyl-group-containing organopolysiloxane described in (7) of the following Table 1, 50.9 parts by mass of dimethylsiloxane (kinematic viscosity of 2 mm²/s) as the oil that is in a liquid state at 25°C, and 0.02 parts by mass of divinyltetramethyldisiloxane solution of 1 mass% chloroplatinic acid as the catalyst for hydrosilylation reaction were added into a reaction vessel, and the mixture was reacted at 70 to 80°C for 2 hours.

Next, the entire amount of the obtained reaction product was sufficiently kneaded with 152.7 parts by mass of the dimethylsiloxane (kinematic viscosity of 2 mm²/s) by using a triple-roller to obtain a smooth paste-like silicone composition having a penetration of 273 in a reactant/liquid oil = 20/80 mass ratio. There was no uncomfortable odor, and no change in properties was observed on the next day. At this time, content ratio, in the paste-like silicone composition, of octamethylcyclotetrasiloxane and decamethylcyclopentasiloxane were each less than 100 ppm, and that of dodecamethylcyclohexasiloxane was 180 ppm.

### Example 3

8.0 parts by mass of the organohydrogenpolysiloxane described in (3) of the following Table 1, 96.5 parts by mass of the alkenyl-group-containing organopolysiloxane described in (9) of the following Table 1, 0.03 parts by mass of divinyltetramethyldisiloxane solution of 1 mass% chloroplatinic acid as the catalyst for hydrosilylation reaction were added into a reaction vessel, and the mixture was reacted at 70 to 80°C for 2 hours.

Next, the entire amount of the obtained reaction product was sufficiently kneaded with 185.8 parts by mass of isododecane as the oil that is in a liquid state at 25°C, using a triple-roller, to obtain a smooth paste-like silicone composition having a penetration of 318 in a reactant/liquid oil = 36/64 mass ratio. There was no uncomfortable odor, and no change in properties was observed on the next day. At this time, content ratio, in the paste-like silicone composition, of octamethylcyclotetrasiloxane, decamethylcyclopentasiloxane, and dodecamethylcyclohexasiloxane were each less than 100 ppm.

### Example 4

7.2 parts by mass of the organohydrogenpolysiloxane described in (5) of the following Table 1, 21.5 parts by mass of the alkenyl-group-containing organopolysiloxane described in (9) of the following Table 1, 114.6 parts by mass of diphenylsiloxy phenyl trimethicone (labeled name; KF-56A, manufactured by Shin-Etsu Chemical Co., Ltd.) as the oil that is in a liquid state at 25°C, 0.02 parts by mass of divinyltetramethyldisiloxane solution of 1 mass% chloroplatinic acid as the catalyst for hydrosilylation reaction were added into a reaction vessel, and the mixture was reacted at 70 to 80°C for 2 hours.

Next, the entire amount of the obtained reaction product was sufficiently kneaded with 143.7 parts by mass of the diphenylsiloxy phenyl trimethicone, using a triple-roller, to obtain a fluid paste-like silicone composition having a viscosity of 78 Pa·s in a reactant/liquid oil = 10/90 mass ratio. There was no uncomfortable odor, and no change in properties was observed on the next day. At this time, content ratio, in the paste-like silicone composition, of octamethylcyclotetrasiloxane, decamethylcyclopentasiloxane, and dodecamethylcyclohexasiloxane were each less than 100 ppm.

### Example 5

8.6 parts by mass of the organohydrogenpolysiloxane described in (4) of the following Table 1, 103.1 parts by mass of the alkenyl-group-containing organopolysiloxane described in (10) of the following Table 1, and 0.03 parts by mass of divinyltetramethyldisiloxane solution of 1 mass% chloroplatinic acid as the catalyst for hydrosilylation reaction were added into a reaction vessel, and the mixture was reacted at 70 to 80°C for 2 hours.

26.4 parts by mass of the obtained reaction product was sufficiently kneaded with 46.9 parts by mass of isododecane as the oil that is in a liquid state at 25°C using a triple-roller, to obtain a paste-like silicone composition having a penetration of 318 in a reactant/liquid oil = 36/64. There was uncomfortable odor slightly. However, properties on the next day of the obtained composition were smooth paste, and no plasticization return was confirmed. At this time, content ratio, in the paste-like silicone composition, of octamethylcyclotetrasiloxane, decamethylcyclopentasiloxane, and dodecamethylcyclohexasiloxane were 1,480 ppm, 1,960 ppm, and 710 ppm respectively.

### Comparative Example 1

45.2 parts by mass of the organohydrogenpolysiloxane described in (2) of the following Table 1, 11.0 parts by mass of the alkenyl-group-containing organopolysiloxane described in (8) of the following Table 1, 56.3 parts by mass of the dimethylsiloxane (kinematic viscosity of 2 mm²/s) as the oil that is in a liquid state at 25°C, and 0.02 parts by mass of divinyltetramethyldisiloxane solution of 1 mass% chloroplatinic acid as the catalyst for hydrosilylation reaction were added into a reaction vessel, and the mixture was reacted at 70 to 80°C for 2 hours.

60.0 parts by mass of the obtained reaction product was sufficiently kneaded with 89.9 parts by mass of the dimethylsiloxane (kinematic viscosity of 2 mm²/s) using a triple-roller to obtain a paste-like silicone composition having a penetration of 307 in a reactant/liquid oil = 20/80 mass ratio. There was uncomfortable odor, and on the next day the compound was cured and plasticization return was confirmed. At this time, content ratio, in the paste-like silicone composition, of octamethylcyclotetrasiloxane, decamethylcyclopentasiloxane, and dodecamethylcyclohexasiloxane were 170 ppm, 2,800 ppm, and 1,900 ppm respectively.

Organopolysiloxane used in Examples 1 to 5 and Comparative Example 1 are shown in the following Table 1, and summarized results of Examples 1 to 5 and Comparative Example 1 are shown in the following Table 2.

**[Table 1]**

| | low molecular cyclic siloxane (ppm) | | | Structural formula |
|---|---|---|---|---|
| | D4 | D5 | D6 | |
| (1) | <100 | <100 | <100 | 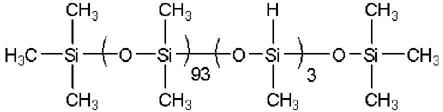 |
| (2) | 440 | 16,450 | 10,150 | |
| (3) | <100 | 250 | 170 | 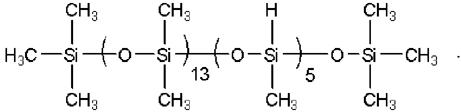 |
| (4) | 16,160 | 7,640 | 2,040 | |
| (5) | <100 | 550 | 1,780 | 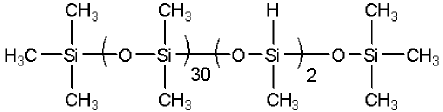 |
| (6) | <100 | <100 | 140 | 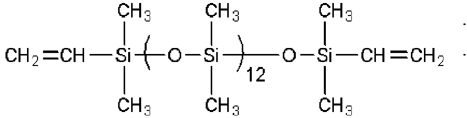 |
| (7) | <100 | 600 | 1,830 | |
| (8) | 3,620 | 7,850 | 5,520 | |
| (9) | <100 | <100 | <100 | 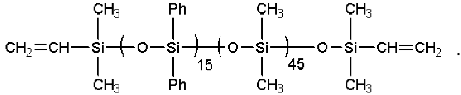 |
| (10) | 2,620 | 4,280 | 1,770 | |

In determining content ratio of cyclic siloxane, samples of organopolysiloxane (1) to (10) are used.
D4: octamethylcyclotetrasiloxane
D5: decamethylcyclopentasiloxane
D6: dodecamethylcyclohexasiloxane

**[Table 2]**

| | Viscosity/ Penetration | Low molecular siloxane in composition (ppm) | | | Odor | plasticization return |
|---|---|---|---|---|---|---|
| | | D4 | D5 | D6 | | |
| Example 1 | Penetration 270 | <100 | <100 | 100 | Absent | Absent |
| Example 2 | Penetration 273 | <100 | <100 | 180 | Absent | Absent |
| Example 3 | Penetration 318 | <100 | <100 | <100 | Absent | Absent |
| Example 4 | Viscosity 78 Pa·s | <100 | <100 | <100 | Absent | Absent |
| Example 5 | Penetration 318 | 1480 | 1960 | 710 | Somewhat | Absent |
| Comparative Example 1 | Penetration 307 | 170 | 2800 | 1900 | Present | Present |

In determining content ratio of cyclic siloxane, samples of paste-like silicone compositions of Examples 1 to 5 and Comparative Example 1.
D4: octamethylcyclotetrasiloxane
D5: decamethylcyclopentasiloxane
D6: dodecamethylcyclohexasiloxane

As Examples 1 to 5 in Table 2 shows, the inventive paste-like silicone composition has small changes over time such as plasticization return and emits less uncomfortable odor, and is stable. Especially, in Examples 1 to 4, there is no uncomfortable odor.

On the other hand, in Comparative Example 1 where the content ratio of any one of octamethylcyclotetrasiloxane, decamethylcyclopentasiloxane, and dodecamethylcyclohexasiloxane is more than 2,000 ppm, the paste-like silicone composition had changes over time such as plasticization return and uncomfortable odor, and was not stable.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A paste-like silicone composition comprising:
(A) crosslinked organopolysiloxane; and
(B) oil in a liquid state at 25°C,
wherein octamethylcyclotetrasiloxane, decamethylcyclopentasiloxane, and dodecamethylcyclohexasiloxane contained in the paste-like silicone composition are each less than 2,000 ppm,
and the component (A) comprises a hydrosilylated reaction product of:
(A-1) organohydrogenpolysiloxane having at least two hydrogen atoms bonded to a silicon atom per molecule; and
(A-2) alkenyl group-containing organopolysiloxane having at least two alkenyl groups having 2 to 10 carbon atoms and being bonded to a silicon atom per molecule.

2. The paste-like silicone composition according to claim 1,
wherein, the component (A-1) is organohydrogenpolysiloxane represented by the following general formula (1), 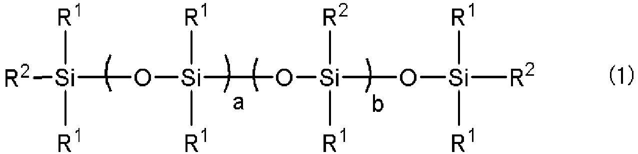 wherein R¹ independently represents a group selected from the group consisting of an alkyl group having 1 to 30 carbon atoms, an aryl group having 6 to 30 carbon atoms, and an aralkyl group having 7 to 30 carbon atoms, R² independently represents a group selected from the group consisting of the R¹ and a hydrogen atom provided that two or more R² in one molecule are hydrogen atoms, "a" is an integer satisfying 3≤a≤100,000, and "b" is an integer satisfying 1≤b≤50.

3. The paste-like silicone composition according to claim 1 or 2,
wherein, the component (A-2) is alkenyl group-containing organopolysiloxane represented by the following general formula (2), 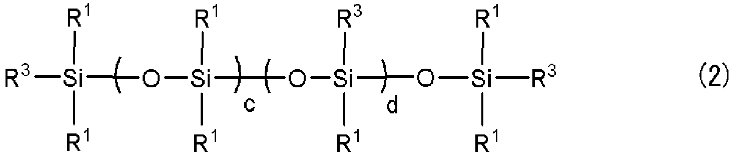 wherein R¹ independently represents a group selected from the group consisting of an alkyl group having 1 to 30 carbon atoms, an aryl group having 6 to 30 carbon atoms, and an aralkyl group having 7 to 30 carbon atoms, R³ independently represents a group selected from the group consisting of the R¹ and an alkenyl group having 2 to 10 carbon atoms provided that two or more R³ in one molecule are alkenyl groups, "c" is an integer satisfying 3≤c≤100,000, and "d" is an integer satisfying 0≤d≤50.

4. The paste-like silicone composition according to claim 3,
wherein, "d" in the general formula (2) satisfies d=0.

5. A method for producing the paste-like silicone composition according to any one of claims 1 to 4, comprising a step of obtaining the component (A) by subjecting the components (A-1) and (A-2) to hydrosilylation reaction in the presence of a catalyst for hydrosilylation reaction, wherein the components (A-1) and (A-2) contain octamethylcyclotetrasiloxane, decamethylcyclopentasiloxane, and dodecamethylcyclohexasiloxane, and each content ratio is 2,000 ppm or less in the components (A-1) and (A-2) respectively.

6. The method for producing the paste-like silicone composition according to claim 5,
wherein the hydrosilylation reaction is carried out in the presence of 0.01 to 100 mass% of component (B) blended in the paste-like silicone composition.

7. Cosmetics contains the paste-like silicone composition according to any one of claims 1 to 4.
